# EUROPÄISCHE PATENTSCHRIFT

(11) **EP 2 286 850 B1**
(45) Veröffentlichungstag und Bekanntmachung des Hinweises auf die Patenterteilung: **20.03.2013**
(21) Anmeldenummer: 10008159.5
(22) Anmeldetag: 05.08.2010
(51) Int. Cl.: A61M 1/16

(54) **Versorgungseinrichtung für Dialysegeräte**
Supply unit for dialysis machines
Dispositif d'alimentation pour appareils de dialyse

(30) Priorität: 22.08.2009 DE 102009038571
(43) Veröffentlichungstag der Anmeldung: 23.02.2011
(73) Patentinhaber: Völker, Manfred, 63825 Blankenbach (DE)
(72) Erfinder: Völker, Manfred, 63825 Blankenbach (DE)
(74) Vertreter: Flosdorff, Jürgen

(56) Entgegenhaltungen:
- DE-A1- 3 320 416
- DE-A1- 19 809 945
- DE-U1- 8 909 542
- US-A1- 2005 161 562

## Beschreibung

Die Erfindung betrifft eine hydraulische Versorgungseinrichtung für Dialysegeräte, mit einer Permeatleitung und wenigstens einer Konzentratleitung zum Zuführen von Permeat und Dialysekonzentrat(en) zu einem Dialysegerät, die dort zu Dialysierflüssigkeit gemischt werden, und mit einer Abwasserleitung zum Abführen von Abwasser aus dem Dialysegerät, wobei das Abwasser in einen Abfluss abgeleitet wird.

Die Permeatleitung führt von einem Umkehrosmosegerät erzeugtes Permeat mehreren in Reihe angeschlossenen Dialysegeräten zu, denen über wenigstens eine Konzentratleitung, meist drei Konzentratleitungen - entweder über drei Kupplungen oder über einen Konzentratauswahlschalter - außerdem das Konzentrat zugeführt wird, das zusammen mit dem Permeat vorzugsweise in den jeweiligen Dialysegeräten zu Dialysierflüssigkeit gemischt und dem Dialysator zugeführt wird, von dem hier allgemein als Abwasser bezeichnete, mit Harnstoffen etc. angereicherte Dialysierflüssigkeit aus dem Dialysegerät abgeführt und einem Abfluss zugeführt wird.

Ein herkömmlicher Abwasserablauf hat erhebliche Nachteile: Er spritzt, plätschert, was zu erheblichen Geräuschbelästigungen der Patienten führt, er riecht unangenehm und ist unhygienisch, neigt zur Bildung von Rückständen, und eine Probeentnahme in Form von papierförmigen Stäbchen zum Beispiel zum Test von chemischen Rückständen nach einer Desinfektion des angeschlossenen Gerätes mit Teststäbchen ist schwierig.

DE 33 20 416 A1 und DE 89 09 542 U1 befassen sich mit einer gemeinsamen Entsorgung für mehrere in einer Dialysestation angeordnete Dialysegeräte. Hierzu wird ein Installationskanal offenbart, der neben zwei Flüssigkeitszuleitungen eine Abflussleitung enthält, in die die Abwasserleitungen der jeweiligen Dialysegeräte einmünden. Diese Abflussleitungen sind mit einem Gefälle versehen, da die Entsorgungsflüssigkeit drucklos in das Abwassersystem geführt wird.

Das Abwasser der einzelnen Dialysegeräte fließt durch Abwasserschläuche, die an Anschlüsse des Installationskanals angekuppelt sind, von wo jeweils ein Fallrohr in die Abflussleitung einmündet.

Der vorliegenden Erfindung liegt nach einem ersten Aspekt die Aufgabe zugrunde, für den Abwasserablauf eine bessere Lösung zu finden, die die obigen Nachteile ganz oder wenigstens teilweise beseitigt.

Die herkömmliche Versorgung von Dialysegeräten über eine Permeatleitung und wenigstens eine Konzentratleitung erfordert einen hohen Installationsaufwand und ist damit teuer, wenn die Leitungen zum Beispiel an einer Zimmerwand angebracht und aus optischen Gründen durch vorgehängte Wandabschnitte überdeckt sind. Dies nimmt auch einen erheblichen Raum in Anspruch.

Nach einem weiteren Aspekt liegt der vorliegenden Erfindung die Aufgabe zugrunde, eine kompakte und kostengünstige Versorgungseinrichtung anzugeben, die leicht zu installieren ist.

Die Erfindung ist in Anspruch 1 definiert.

Nach dem ersten Aspekt der Erfindung ist vorgesehen, dass die Abwasserleitung zu einem im wesentlichen trichterförmigen Ablaufbehälter führt, in den das Abwasser im freien Fall fällt und dabei auf einen schrägen Wandabschnitt des Ablaufbehälters auftrifft. Der Winkel des wenigstens einen schrägen Wandabschnitts zur Horizontalen sollte dabei mindestens 45 Grad betragen.

Der freie Auslauf des Abwassers in den trichterförmigen Behälter vermeidet zuverlässig eine Rückkontamination der Abwasserleitung aus dem Abfluss.

Da das Abwasser im freien Fall auf einen zugehörigen schrägen Wandabschnitt des Ablaufbehälters auftrifft, entstehen hierbei praktisch keine von den Patienten wahrnehmbare Geräusche, wozu beiträgt, dass der Ablaufbehälter aus einem Trichter, einem oberen Deckel und einer schwenkbaren Klappe zu einem geschlossenen Gehäuse zusammengesetzt ist. Die schwenkbare Klappe, die vorzugsweise in einer Seitenwand des Ablaufbehälters angeordnet und normalerweise geschlossen ist, ermöglicht auf einfache Weise eine Probeentnahme aus dem Ablaufbehälter mittels Teststäbchen. Das ansonsten geschlossene Gehäuse verhindert zuverlässig den Austritt von unangenehmen Gerüchen ins Patientenzimmer.

Mit besonderem Vorteil wird vorgeschlagen, dass durch den Deckel des Ablaufbehälters wenigstens eine Abwasser-Einlauftülle führt, die ins Innere des Behälters ragt. Dabei können auch mehrere Abwasser-Einlauftüllen, beispielsweise vier Tüllen vorgesehen sein, die mit den Abwasserleitungen unterschiedlicher Geräte verbunden sind.

Weiter wird vorgeschlagen, dass jede Abwasser-Einlauftülle einen unteren Rand hat, der so geformt ist, dass der Abwasserstrahl glatt vertikal, im wesentlichen ohne Verwirbelung, nach unten aus der Einlauftülle austritt, und zwar auch dann ohne Verwirbelung, wenn nur ein geringer Durchfluss durch die Einlauftülle herrscht. Hierzu wird vorgeschlagen, dass der untere Randabschnitt der Einlauftülle wenigstens einen spitzen Vorsprung aufweist, vorzugsweise sind zwei diametral gegenüberliegende spitze Vorsprünge vorgesehen, zwischen denen der Randabschnitt der Einlauftülle in einer konkaven Krümmung verläuft. Hierdurch wird ein guter Strahlabriss hervorgerufen, so dass das frei fallende Abwasser zuverlässig auf den oder die unter den Einlauftüllen angeordneten schrägen Wandabschnitten auftrifft, was zu einem praktisch geräuschfreien Abfließen des Abwassers führt.

Der Trichter ist vorzugsweise ebenso wie der Deckel und die um eine obere horizontale Achse schwenkbare Klappe ein Kunststoffspritzteil. Am unteren Endbereich des Trichters kann außen ein Gewinde angeformt sein, auf das ein Abflussrohr bzw. ein flexibler Abflussschlauch aufschraubbar ist. Im Inneren des unteren Endbereichs können sich Stege über den Querschnitt erstrecken, auf die eine Siphonreinigungstablette gelegt werden kann, die durch die geöffnete Klappe einbringbar ist.

Der Trichter besteht zweckmäßigerweise aus einem oberen quaderförmigen Abschnitt, dessen eine Längswand durch die schwenkbare Klappe gebildet ist, und einem unteren, mit schrägem Übergang eingeschnürten, im wesentlichen trichterförmigen Abschnitt, auf dessen schräge Wände das fallende Abwasser hauptsächlich auftrifft. An Stelle des unteren Gewindes kann auch ein unterer Steckanschluss zur Befestigung an eine weiterführende Standardrohrleitung, bzw. Schlauchleitung oder an einem Siphon vorgesehen sein. Der Trichter ist optisch ansprechend und hat glatt ineinander übergehende innere Wandabschnitte, an denen sich kaum Rückstände anlagern können, womit der Ablaufbehälter sehr hygienisch ist.

Nach einem weiteren Aspekt der vorliegenden Erfindung ist in die Permeatleitung wenigstens ein Permeatblock eingefügt, während in die Konzentratleitungen entsprechend wenigstens ein Konzentratblock oder Konzentratwahlschalter eingesetzt ist. Diese beiden Blöcke bestehen vorteilhafterweise aus Kunststoff und haben die zugehörigen Bohrungen, um Abschnitte der Permeatleitung und der Konzentratleitungen zu bilden. Für jedes Dialysegerät kann ein Permeatblock und ein Konzentratblock vorgesehen sein, wobei die Blöcke auch länger ausgeführt sein können, um beispielsweise mehrere Dialysegeräte eines Patientenzimmers zu versorgen.

Alle Permeatblöcke und Konzentratblöcke sind an ihren Stirnseiten mit Versorgungsanschlüssen versehen, die als Schlauchtüllen- oder Steckanschlüssen ausgebildet sein sollten, und von ihren Längsbohrungen zweigen seitlich Kupplungen oder Schlauchanschlüsse zum Anschluss der zu den Dialysegeräten führenden Schläuchen ab.

Weiter wird mit großem Vorteil vorgeschlagen, dass die Permeatleitung die Konzentratleitungen sowie die Permeatblöcke und Konzentratblöcke in einem Versorgungskanal angeordnet sind, aus dessen Seitenwand die Kupplungen bzw. die Anschlüsse für die hydraulischen Versorgungsleitungen herausragen. Dieser Versorgungskanal hat zweckmäßigerweise eine rechteckige Querschnittsform und ist bevorzugt aus einem etwa C-förmigen Kanalelement und einer ebenen Seitenwand zusammengesetzt, aus der die Anschlüsse für das Dialysegerät herausragen. Der Versorgungskanal kann jedoch eine den Installations- und Anschlussanforderungen andere angepasste Form haben. Dieser Versorgungskanal kann beispielsweise an einer Zimmerwand befestigt sein, wo er kaum in Erscheinung tritt, oder er kann an Ständern in der Nähe der Dialysegeräte gehalten sein. Ebenso ist es möglich den Versorgungskanal an einen an der Wand befestigten Schwenkarm zu montieren, um den hygienischen und räumlichen Notwendigkeiten noch besser zu entsprechen. Dabei ist eine vertikale oder horizontale Montage der Versorgungseinheit möglich.

Die Permeatblöcke und die Konzentratblöcke sind vorteilhafterweise paarweise übereinander in dem Versorgungskanal angeordnet und greifen dabei mit schwalbenschwanzförmigen Abschnitten ineinander ein. Anstelle des einen Konzentratblockes mit bis zu drei Konzentratanschlüssen ist es möglich einen Konzentratwahlschalter zu integrieren, der es dem Anwender einfach, ohne Umzukuppeln ermöglicht, das jeweils benötigte Konzentrat für das Dialysegerät auszuwählen.

Weiter wird vorgeschlagen, dass der Deckel des Trichters an der Unterseite des Versorgungskanals angebracht wird, wobei die Abwasser-Einlauftüllen ins Innere des Versorgungskanals ragen, und dass der Permeatblock wenigstens eine von der Permeatbohrung getrennte Abwasserleitungsbohrung mit einer durch die Seitenwand des Kanals führenden Abwasserkupplung aufweist, wobei die Abwasserleitungsbohrung mit der wenigstens einen Abwasser-Einlauftülle verbunden ist.

Die oben beschriebene hydraulische Versorgungseinrichtung kann außerdem mit einem zweckmäßigerweise daneben oder oberhalb liegenden zur elektrischen Versorgung und Kommunikation des Verbrauchers kombiniert sein.

Die erfindungsgemäße Versorgungseinrichtung ist kostengünstig herstellbar und mit geringem Aufwand zu installieren. Sie hat eine ansprechende äußere Gestaltung und muss daher nicht mit vorgeschalteten Blendwänden in einem Patientenzimmer überdeckt werden.

Weitere Einzelheiten der Erfindung ergeben sich aus der nachfolgenden Beschreibung einer bevorzugten Ausführungsform des Abwasser-Ablaufbehälter und eines Versorgungsmoduls. Dabei zeigen:
- Figur 1: eine perspektivische Ansicht eines Abwasser-Ablaufbehälters;
- Figur 2: einen Längsschnitt durch den Behälter der Figur 1;
- Figur 3: einen Querschnitt durch den oberen Teil des Behälters der Figur 1;

- Figur 4: eine teilweise geschnittene Seitenansicht des Abwasser-Ablaufbehälters mit einem Wandbefestigungsteil;
- Figur 5: eine perspektivische Ansicht eines Versorgungsrnoduls mit Ablaufbehälter mit entfernter vorderer Kanalwand;
- Figur 6: eine Darstellung ähnlich Figur 5, jedoch mit seitlich geschlossenem Kanal,
- Figur 7: einen Vertikalschnitt durch das Versorgungsmodul gemäß Figur 6 und
- Figur 8: ein an einem Gelenkarm befestigtes Versorgungsmodul.

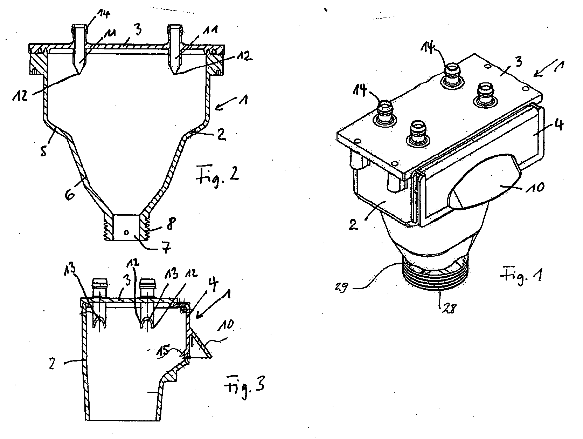

In den Figuren 1 bis 4 ist ein Ablaufbehälter 1 dargestellt, in den das von einem nicht dargestellten Dialysegerät kommende Abwasser einem Abfluss zugeführt wird. Der Ablaufbehälter 1 ist aus drei Behälterteilen zusammengesetzt, nämlich einem Trichter 2, einem die Oberseite des Trichters 2 verschließenden Deckel 3 und einer Klappe 4, die um eine obere Längsachse nach außen schwenkbar ist. Diese drei Behälterteile sind im Spritzgussverfahren aus Kunststoff hergestellt.

Der Trichter 2 hat einen oberen, im wesentlichen quaderförmigen Abschnitt, dessen eine Längswand die Klappe 4 bildet. Der quaderförmige Abschnitt geht über nach innen verlaufende Wandabschnitte 5 in den etwa trichterförmigen unteren Abschnitt 6 über, der sich zum unteren Ende hin in Schrägen verengt, deren Neigung zur Horizontalen größer als 45 Grad ist. Der untere zylindrische Endabschnitt 7 ist mit einem Außengewinde 8 versehen, auf das ein Standard-Abflussrohr schraubbar ist.

Die Klappe 4 enthält an ihren oberen seitlichen Enden angeformte Scharnierkugeln 9, die in zugehörige Löcher der Trichterwand eingerastet sind, so dass die Klappe 4 an einem angeformten Handgriff 10 bequem nach oben schwenkbar ist. Hierdurch kann beispielsweise eine Siphonreinigungstablette auf sternförmig den unteren Endabschnitt 7 überbrückende Stege (nicht dargestellt) gelegt werden, oder es können Teststäbchen ins Innere des Ablaufbehälters eingeführt werden.

Die Übergänge der einzelnen Wandabschnitte sind glatt gerundet, so dass sich praktisch keine Ablagerungen an den Innenwänden absetzen können.

Durch den Deckel 3 verlaufen vier Abwasser-Einlauftüllen, die innerhalb des Ablaufbehälters 1 in einer besonderen Randkontur enden, nämlich zwei diametral gegenüberliegenden Spitzen 12 und dazwischen liegenden konkaven Ausschnitten 13 . Hierdurch wird ein glatter Flüssigkeitsstrahlabriss ohne Verwirbelung selbst bei geringem Wasserfluss erreicht, so dass das Abwasser zuverlässig und glatt auf die schräge Wand des trichterförmigen Abschnitts 6 des Ablaufbehälters 1 auftrifft und praktisch geräuschlos abfließt. Die Abwasser-Einlauftüllen 11 sind an der Oberseite des Deckels 3 mit Schlauchtüllenanschlüssen 14 versehen. In die Schlauchtüllenanschlüsse sind zusätzlich Einschnürungen für die Aufnahme von Dichtringen mit eingebracht.

Am unteren Endabschnitt der Klappe 4 ist eine nach innen ragende Tropfkante 15 ausgebildet, zur Ableitung von Schwitzwasser.

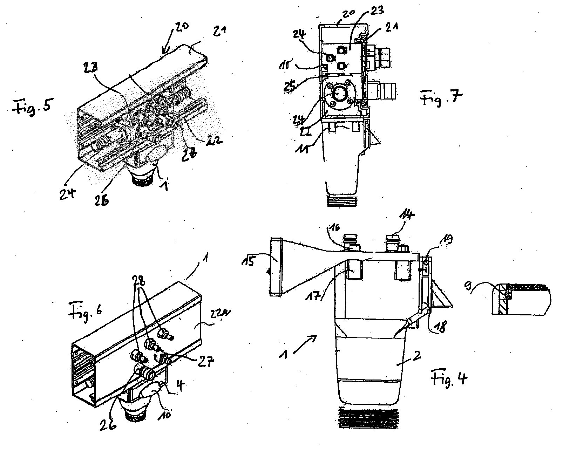

Wie Figur 4 zeigt, kann an dem Ablaufbehälter 1 ein Wandbefestigungsteil 15 vorgesehen sein. Mit Befestigungsschrauben 16 die in Gewindemutteraufnahmen 17 sitzende Gewindemutter eingeschraubt sind wird der Deckel 3 am Trichterkorpus 2 befestigt. In dieser Figur ist außerdem eine Nut 18 für den Frontrahmeneinbau und eines der beiden seitlichen Löcher 19 für den Eingriff der Scharnierkugeln 9 zu erkennen.

Die Figuren 5 bis 7 zeigen einen Versorgungskanal 20, in dem eine Permeatleitung und Konzentratleitungen zur Versorgung von angeschlossenen Dialysegeräten untergebracht sind. Der Versorgungskanal 20 ist aus einem wesentlichen C-förmigen Kanalabschnitt 21 und einem ebenen Seitenabschnitt 22 zu einem geschlossenen Rechteck zusammengesetzt.

Die Figuren zeigen einen Permeatblock 22 und einen Konzentratblock 23, die in die Permeatleitung und die Konzentratleitungen eingeschaltet sind, wozu an den Stirnseiten des Permeatblocks 22 Permeatversorgungsanschlüsse 24 und an den Stirnseiten des Konzentratsblocks 23 Konzentrat-Versorgungsanschlüsse 24 angeordnet sind. Der Permeatblock 22 und der Konzentratblock 23 bestehen aus im wesentlichen quaderförmigen Kunststoffblöcken mit entsprechenden durchgehenden Bohrungen, wobei die beiden Blöcke 22 und 23 durch eine Schwalbenschwanzverbindung 25 zusammengesetzt sind.

Der Permeatblock 22 enthält außerdem eine Abwasserbohrung, die getrennt von der Permeatbohrung verläuft und in die eine Abwasserkupplung 26 seitlich einmündet. An der Unterseite des Kanals 20 ist der Deckel 3 des Ablaufbehälters 1 befestigt, wobei seine Einlauftüllen 14 mit der Abwasserbohrung des Permeatblocks 22 in Verbindung stehen.

Die in Längsrichtung durch den Permeatblock 22 verlaufende Permeatbohrung steht mit einer Permeatkupplung 27 in Verbindung, während Konzentratkupplungen 28 mit den Bohrungen des Konzentratblocks 23 in Verbindung stehen. An diese Kupplungen sind Schläuche anschließbar, die zu dem jeweiligen Dialysegerät führen.

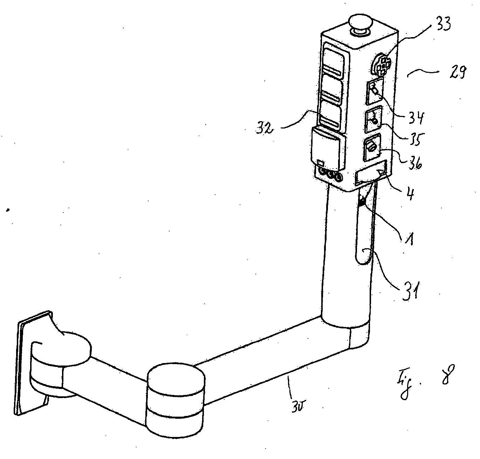

In Figur 8 ist ein Versorgungsmodul 29 dargestellt, das von einem Gelenkarm 30 gehalten ist, der beispielsweise an der Wand eines Patientenzimmers befestigt sein kann. Gegenüber den Figuren 5 bis 7 wurden die Konzentratkupplungen 28 durch einen Konzentratwahlschalter 33 ersetzt. Sowohl die Permeatkupplung 27 als auch die Konzentratkupplung 28 werden bei dieser Ausführung direkt am Dialysegerät gekuppelt. Die angezeigten Kupplungspositionen 34, 35, 36 werden bei diskonnektierten Dialysegerät als Parkstation für die freien Kupplungen genutzt.

Von der Unterseite des Moduls 29 steht ein Ablaufbehälter 1 vor, dessen Unterseite durch einen Schlitz 31 in dem Befestigungsarm 30 zugänglich ist. Die zu dem Modul 29 führenden Versorgungsschläuche können durch das Innere des Gelenkarms 30 verlaufen. Das Versorgungsmodul 29 ist für ein zugehöriges Dialysegerät in dessen Nähe angeordnet.

Es kann zusätzlich elektrische Leitungen mit an einer weiteren Außenwand des Moduls 29 angeordneten Schaltern 32 etc. enthalten.

Es wird betont, dass die Erfindung nicht auf die beschriebenen und dargestellten Ausführungsformen beschränkt ist. Vielmehr sind alle offenbarten Merkmale auf jede sinnvolle Weise einzeln miteinander kombinierbar.

## Patentansprüche

1. Versorgungseinrichtung für Dialysegeräte, mit einer Permeatleitung und wenigstens einer Konzentratleitung zum Zuführen von Permeat und Dialysekonzentrat(en) zu einem Dialysegerät, die zu Dialysierflüssigkeit gemischt werden, und mit einer Abwasserleitung zum Abführen von Abwasser aus dem Dialysegerät,
**dadurch gekennzeichnet,**
**dass** die Abwasserleitung zu einem im wesentlichen trichterförmigen Ablaufbehälter (1) führt, in den das Abwasser im freien Fall fällt und dabei auf einen schrägen Wandabschnitt (6) des Ablaufbehälters (1) auftrifft, und
**dass** der Ablaufbehälter (1) aus einem Trichter (2), einem oberen Deckel (3) und einer schwenkbaren Klappe (4) zusammengesetzt ist.

2. Versorgungseinrichtung nach Anspruch 1,
**dadurch gekennzeichnet,**
**dass** durch den Deckel (3) wenigstens eine Abwasser-Einlauftülle (11) führt.

3. Versorgungseinrichtung nach Anspruch 2,
**dadurch gekennzeichnet,**
**dass** die wenigstens eine Abwasser-Einlauftülle (11) einen unteren Randabschnitt mit wenigstens einem spitzen Vorsprung (12) aufweist.

4. Versorgungseinrichtung nach Anspruch 3,
**dadurch gekennzeichnet,**
**dass** der Randabschnitt zwei gegenüberliegende spitze Vorsprünge (12) aufweißt, zwischen denen der Randabschnitt in konkaven Randkanten (13) endet.

5. Versorgungseinrichtung nach einem der Ansprüche 1 bis 4,
**dadurch gekennzeichnet,**
**dass** im unteren Endbereich des Trichters (2) quer über den Innenraum verlaufende Stege angeformt sind, auf die eine Siphonreinigungstablette legbar ist.

6. Versorgungseinrichtung nach Anspruch 1,
**dadurch gekennzeichnet,**
**dass** in die Permeatleitung und in die wenigstens eine Konzentratleitung wenigstens ein Permeatblock (22) und wenigstens ein Konzentratblock (23) eingefügt sind, die die zugehörigen Bohrungen aufweisen.

7. Versorgungseinrichtung nach Anspruch 6,
**dadurch gekennzeichnet,**
**dass** alle Permeatblöcke (22) und alle Konzentratblöcke (23) mit Versorgungsanschlüssen (24), die als Schlauchtüllen- oder Steckanschlüsse ausgebildet sind, und mit Kupplungen (26, 27, 28) versehen sind.

8. Versorgungseinrichtung nach Anspruch 6 oder 7,
**dadurch gekennzeichnet,**
**dass** die Permeatleitung, die Konzentratleitungen sowie die Permeatblöcke (22) und Konzentratblöcke (23) in einem Versorgungskanal (20) angeordnet sind, aus dessen Seitenwand (22a) die Kupplungen (26, 27, 28) herausragen.

9. Versorgungseinrichtung nach Anspruch 8,
**dadurch gekennzeichnet,**
**dass** der Deckel (3) des Ablaufbehälters (1) an der Unterseite des Versorgungskanals (20) angebracht ist und dass der Permeatblock (22) wenigstens eine getrennte Abwasserleitungsbohrung mit einer durch die Seitenwand (22a) des Kanals (20) führender Abwasserkupplung (26) aufweist, wobei die A bwasserleitungsbohrung mit der wenigstens einen Abwasser-Einlauftülle (11) verbunden ist.

## Claims

1. A supply device for dialysis machines with a permeate line and at least one concentrate line for supplying permeate and dialysis concentrate(s) to a dialysis machine, which are mixed into the dialysis liquid, and with a liquid waste line for discharging liquid waste out of the dialysis machine, **characterised in that** the liquid waste line leads to a substantially hopper-shaped discharge container (1), into which the liquid waste falls in free fall and thereby impinges on an oblique wall section (6) of the discharge container (1) and that the discharge container (1) is composed of a hopper (2), an upper lid (3) and a pivotable flap (4).

2. A supply device as claimed in claim 1, **characterised in that** at least one liquid waste inlet nozzle (11) extends through the lid (3).

3. A supply device as claimed in claim 2, **characterised in that** the at least one liquid waste inlet nozzle (11) has a lower edge section with at least one pointed projection (12).

4. A supply device as claimed in claim 3, **characterised in that** the edge section has two opposing pointed projections (12), between which the edge section terminates in concave outer edges (13).

5. A supply device as claimed in one of claims 1 to 4, **characterised in that** integrally formed in the lower end region of the hopper (2) are webs extending transversely above the interior space, onto which a syphon cleaning tablet may be placed.

6. A supply device as claimed in claim 1, **characterised in that** inserted into the permeate line and into the at least one concentrate line there are at least one permeate block (22) and at least one concentrate block (23), which afford the associated bores.

7. A supply device as claimed in claim 6, **characterised in that** all the permeate blocks (22) and all the concentrate blocks (23) are provided with supply connectors (24), which are constructed in the form of hose, nozzle connections or plug connections, and with couplings (26, 27, 28).

8. A supply device as claimed in claim 6 or 7, **characterised in that** the permeate line, the concentrate lines and the permeate blocks (22) and concentrate blocks (23) are arranged in a supply passage (20), extending out of whose side wall (22a) are the couplings (26, 27, 28).

9. A supply device as claimed in claim 8, **characterised in that** the lid (3) of the discharge container (1) is attached to the underside of the supply passage (20) and that the permeate block (22) has at least one separate liquid waste line bore with a liquid waste coupling (26) extending through the side wall (22a) of the passage (20), wherein the liquid waste line bore is connected to the at least one liquid waste inlet nozzle (11).

## Revendications

1. Dispositif d'alimentation pour appareils de dialyse, comprenant une conduite de perméat et au moins une conduite de concentré servant à amener le perméat et le/les concentrés de dialyse à un appareil de dialyse, lesquels sont mélangés pour obtenir un liquide de dialyse, et comprenant une conduite pour eaux résiduaires servant à évacuer les eaux résiduaires de l'appareil de dialyse,
**caractérisé en ce que**
la conduite pour eaux résiduaires mène à un réservoir de décharge (1) présentant essentiellement une forme d'entonnoir, dans lequel les eaux résiduaires tombent en chute libre pour se heurter à une section de paroi (6) oblique du réservoir de décharge (1), et **en ce que** le réservoir de décharge (1) est composé d'un entonnoir (2), d'un couvercle supérieur (3) et d'un clapet (4) pouvant pivoter.

2. Dispositif d'alimentation selon la revendication 1,
**caractérisé en ce**
**qu'**au moins un embout d'admission pour les eaux résiduaires (11) mène à travers le couvercle (3).

3. Dispositif d'alimentation selon la revendication 2,
**caractérisé en ce**
**que** l'embout d'admission pour les eaux résiduaires (11) au moins au nombre de un présente une section de bordure inférieure dotée au moins d'une partie faisant saillie (12) pointue.

4. Dispositif d'alimentation selon la revendication 3,
**caractérisé en ce**
**que** la section de bordure présente deux parties faisant saillie (12) pointues se faisant face, entre lesquelles la section de bordure se termine en arêtes de bordure (13) concaves.

5. Dispositif d'alimentation selon l'une quelconque des revendications 1 à 4,
**caractérisé en ce**
**que** dans la zone d'extrémité inférieure de l'entonnoir (2) sont formées des entretoises s'étendant de manière transversale au-delà de l'espace intérieur, sur lesquelles peut être posé un plateau de nettoyage de siphon.

6. Dispositif d'alimentation selon la revendication 1,
**caractérisé en ce**
**qu'**au moins un bloc de perméat (22) et au moins un bloc de concentré (23) sont insérés dans la conduite de perméat et dans la conduite de concentré au moins au nombre de une, lesquels blocs présentent les alésages associés.

7. Dispositif d'alimentation selon la revendication 6,
**caractérisé en ce**
**que** tous les blocs de perméat (22) et tous les blocs de concentré (23) sont pourvus de raccords d'alimentation (24), qui sont réalisés sous la forme de raccords à embout flexible ou de raccords enfichables, et de systèmes d'accouplement (26, 27, 28).

8. Dispositif d'alimentation selon la revendication 6 ou 7,
**caractérisé en ce**
**que** la conduite de perméat, les conduites de concentré ainsi que les blocs de perméat (22) et les blocs de concentré (23) sont disposés dans un canal d'alimentation (20), de la paroi latérale (22a) duquel font saillie les systèmes d'accouplement (26, 27, 28).

9. Dispositif d'alimentation selon la revendication 8,
**caractérisé en ce**
**que** le couvercle (3) du réservoir de décharge (1) est placé au niveau du côté inférieur du canal d'alimentation (20), et en ce que le bloc de perméat (22) présente au moins un alésage de conduite pour eaux résiduaires séparé doté d'un système d'accouplement pour eaux résiduaires (26) menant à travers la paroi latérale (22a) du canal (20), sachant que l'alésage de conduite pour eaux résiduaires est relié à l'embout d'admission pour eaux résiduaires (11) au moins au nombre de un.
